# EUROPEAN PATENT SPECIFICATION

(11) **EP 1 568 781 B1**
(45) Date of publication and mention of the grant of the patent: **13.01.2010**
(21) Application number: 05004018.7
(22) Date of filing: 24.02.2005
(51) Int. Cl.: C12P 17/08, C07D 313/00, A61K 31/365, A61P 17/00, A61K 8/49, A61Q 19/08

(54) **Melanogenesis-modulating macrolides assessed by mammalian cell based activity assays**
Makrolide zur Modulierung der Melanogenese identifiziert mit Hilfe eines auf Säugetierzellen basierendenen Aktivitätstests
Macrolides qui modulent la mélanogénèse évalués sur un test d'activité sur de cellules de mammifères

(30) Priority: 25.02.2004 EP 04004261
(43) Date of publication of application: 31.08.2005
(73) Proprietor: BRAIN AG, 64673 Zwingenberg (DE)
(72) Inventor: Grond, Stephanie, 37077 Göttingen (DE); Krohn, Michael, 64653 Lorsch (DE); Dechert, Ute, 64285 Darmstadt (DE); Eck, Jürgen, 64646 Heppenheim (DE)
(74) Representative: Vossius & Partner

(56) References cited:
- WO-A-03/010121
- US-A- 4 873 347
- IMOKAWA G ET AL: "LOSS OF MELANOGENIC PROPERTIES IN TYROSINASES INDUCED BY GLYCOSYLATION INHIBITORS WITHIN MALIGNANT MELANOMA CELLS" CANCER RESEARCH, AMERICAN ASSOCIATION FOR CANCER RESEARCH, BALTIMORE, MD, US, vol. 42, no. 5, 1982, pages 1994-2002, XP008049254 ISSN: 0008-5472
- REGNIER M ET AL: "KERATINOCYTE-MELANOCYTE CO-CULTURES AND PIGMENTED RECONSTRUCTED HUMAN EPIDERMIS: MODELS TO STUDY MODULATION OF MELANOGENESIS" CELLULAR AND MOLECULAR BIOLOGY, CMB ASSOCIATIONS, NOISY-LE-GRAND, FR, vol. 45, no. 7, November 1999 (1999-11), pages 969-980, XP008049252 ISSN: 0145-5680
- MAHALINGAM H ET AL: "Characterization of density-dependent regulation of the tyrosinase gene promoter: role of protein kinase C." EXPERIMENTAL CELL RESEARCH. 25 NOV 1997, vol. 237, no. 1, 25 November 1997 (1997-11-25), pages 83-92, XP002343327 ISSN: 0014-4827
- IVANOVA VENETA ET AL: "Malonylniphimycin: Macrolide antibiotic from Streptomyces hygroscopicus B-7: Physico-chemical properties and structure elucidation" JOURNAL OF ANTIBIOTICS (TOKYO), vol. 50, no. 11, November 1997 (1997-11), pages 965-969, XP008048850 ISSN: 0021-8820
- IVANOVA VENETA ET AL: "Dihydroniphimycin: New polyol macrolide antibiotic produced by Streptomyces hygroscopicus 15. Isolation and structure elucidation" JOURNAL OF ANTIBIOTICS (TOKYO), vol. 53, no. 6, June 2000 (2000-06), pages 627-632, XP008049115 ISSN: 0021-8820
- GRABLEY S ET AL: "SECONDARY METABOLITES BY CHEMICAL SCREENING II. AMYCINS A AND B, TWO NOVEL NIPHIMYCIN ANALOGS ISOLATED FROM A HIGH PRODUCER STRAIN OF ELAIOPHYLIN AND NIGERICIN" JOURNAL OF ANTIBIOTICS, JAPAN ANTIBIOTICS RESEARCH ASSOCIATION, TOKYO, JP, vol. 43, no. 6, 1990, pages 639-647, XP008048869 ISSN: 0021-8820
- STEFANNELI STEFANIA ET AL: "Inhibitors of type-I interleukin-1 receptor from microbial metabolites" JOURNAL OF ANTIBIOTICS (TOKYO), vol. 50, no. 6, 1997, pages 484-489, XP008048860 ISSN: 0021-8820

## Description

The present invention relates to a compound having activity in a cell-based tyrosinase assay, obtainable or obtained by (a) culturing *Streptomyces hygroscopicus subsp. hygroscopicus* (DSMZ deposit number DSM 40578); (b) separating the medium containing the compound from the bacterial cells; (c) solid phase extraction of said medium, thereby obtaining a crude extract; (d) performing gel filtration of said crude extract; (e) taking a gel filtration fraction exhibiting activity in said assay; (f) performing HPLC of said fraction exhibiting activity in said assay; (g) taking one or more HPLC fractions exhibiting activity in said assay, thereby obtaining said compound; and (h) testing endogenous tyrosinase induction, wherein said assay comprises determining formation of DOPA-quinone from tyrosine or DOPA. Also described herein are methods of cosmetic treatment comprising the administration of a compound represented by the general formula (I) 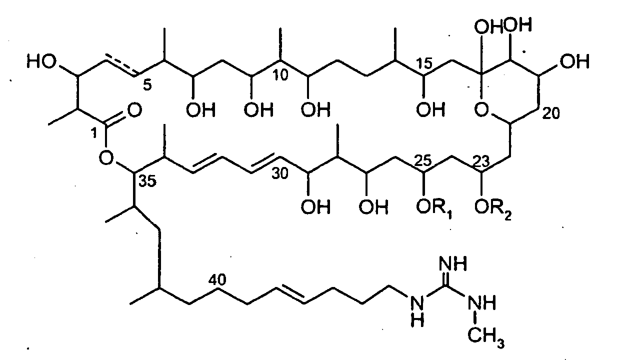 The present invention also relates to uses of said compound as defined in the claims for the preparation of a cosmetic composition. Preferably, said use is a use wherein the application of the cosmetic composition modulates, or, more preferably, brings about the pigmentation of the body part to which said cosmetic composition is applied to.

Skin and hair color is determined by the relative amounts of eumelanin, the brown-black pigment, and pheomelanin, the red-yellow pigment, in epidermal and follicular melanocytes, respectively. Pigmentation of mammalian hair and skin depends on various factors and, in particular, environmental factors (seasons of the year), race, sex and age. The melanocytes are highly differentiated dendritic cells which synthesize melanin by means of specific organelles, the melanosomes, which are, based on their final colour, distinguished as pheo- and eumelanosomes, respectively. These melanosomes progress through a series of defined developmental stages (I-IV) within melanocytes, beginning as unpigmented precursors and ending as a membrane-enclosed bag of melanin. The melanin is later distributed to keratinocytes via the melanocyte dendrites. Within the lumen of melanosomes eumelanin is synthesized from the intermediate dopaquinone by successive hydroxylation, oxidation and carboxylation reactions. In the case of pheomelanin at least one cysteine dependent reduction step (use of cysteinyldopa) is needed.
The synthesis of the key precursor dopaquinone is catalyzed by tyrosinase (monophenol monooxygenase; EC 1.14.18.1) using L-tyrosine as educt for the conversion reaction to dopaquinone via the intermediate dopa (dihydroxyphenylalanine).
Many genes involved in melanogenesis have been identified through comparative genomic studies of mouse coat colour mutations and by the molecular characterisation of human hypopigmentary genetic diseases such as albinism of which the major forms are described as oculocutaneous albinism type 1, 2 and 3 (OCA1 - 3).
A key role in regulation of melanogenesis has been attributed to the melanocortin receptor 1 (MC1-R) which is one out of five members (MC1-R - MC5-R) belonging to the family of G-protein coupled melanocortin receptors. Agonistic ligands of this receptor family are the adrenocorticotropic hormone (ACTH), alpha-melanocortin stimulating hormone (alpha-MSH), beta-MSH, gamma-MSH, corticotropin-like intermediate lobe peptide (CLIP), beta-lipotropin and beta-endorphin. These peptide hormones are all processed postranslationally by various prohormone convertases from a 31 - 36 kD precursor protein, the preprohormone proopiomelanocortin (POMC).
The melanocortin receptors are involved in a variety of physiological aspects and their function is linked to pigmentation in case of MC1-R, steroidogenesis (MC2-R), energy homeostasis (MC3-R), energy homestasis and erectile activity (MC4-R) and sebaceous gland lipid secretion (MC5-R). All of these receptors having alpha-MSH as their preferred ligand with the exception of MC2-R where ACTH is the most potent ligand. For the receptors MC1-R, MC3-R and MC4-R the peptides agouti and agouti related protein (AgRP) are described as antagonistic ligands. The functional diversity of the melanocortin receptors implicates a vast area of application. Thus, novel bioactive compounds which interfere with the endogenous signal transduction of these receptors will have a potential in modulation of skin and hair pigmentation, treatment of obesity, regulation of fat consumption, sexual dysfunction, acne, pain and memory dysfunction, skin cancer and inflammatory deseases. (MacNeil et al. (2002), Samama et al. (2003)).
The melanocyte responds to the peptide hormones alpha-melanocortin stimulating hormone (alpha-MSH) or adrenocorticotropic hormone (ACTH), and, to a lesser extent, to beta-MSH and gamma-MSH through the MC1R G-protein coupled receptor (melanocortin receptor 1) to stimulate melanin production. Presumably this stimulation results from the induction of the second messenger cAMP. It is confirmed that all members of the melanocortin receptor family couple to the adenylyl cyclase/protein kinase A (PKA) intracellular signaling pathway. (Scott et al. (2002), Suzuki et al. (1996)).

The pheomelanosome, containing the tyrosinase, the key enzyme of melanogenesis, produces light red/yellowish melanin, whereas the eumelanosome produces darker melanins via induction of additional TYRP1, TYRP2, SILV enzymes, and the P-protein. Intramelanosomal pH governed by the P-protein may act as a critical determinant of tyrosinase enzyme activity to control the initial step in melanin synthesis or TYRP complex formation to facilitate melanogenesis and melanosomal maturation. Functional correlation of MC1R alleles with more than 30 known variants within skin and hair colour provides evidence that this receptor molecule is a key component underlying normal human pigment variation. Among the approximately 70 genes involved in melanogenesis the tyrosinase, the two tyrosinase-related proteins TRP-1 (an oxidase) and TRP-2 (tautomerase) together with the melanocortin receptor 1 are regarded as the major regulators of mammalian melanogenesis, which is up to now only partially understood.
It has been shown that the beta isoform of the Protein Kinase C (PKC) modulates human melanogenesis through tyrosinase activation. From homologous phosphorylation domains of the cytoplasmatic tail of the tyrosinase as well as of the TRPs it can be concluded that phosphorylation of the C-terminus is involved in the modulation of tyrosinase activity. It is further known from co-cultivation studies that the interplay of melanocytes and keratinocytes affects the level of e. g. the TRPs in melanocytes. However, the regulation of the keratinocyte-melanocyte interactions and the mechanism of melanosome transfer into keratinocytes are not yet understood.

Skin coloring has been of concem to human beings for many years. In particular, the ability to generate a tanned appearance without incurring photodamage due to solar radiation is important to many individuals. There have been several methods proposed to accomplish depigmentation (kojic acid, hydxocione, retinoids), as well as to "tan" the skin without exposure to the sun (dihydroxyacetone and like chemical compounds). Likewise, the ability to remove hyperpigmentation, such as found in age spots, freckles or aging skin generally, or even general body whitening is of interest to individuals desiring a uniform complexion. There are also pigmentation disorders such as, for example, albinism or vitiligo, which is an autoimmune disease characterized by white patches on the skin, that are desirable to treat. In spite of existing artificial dyeing techniques/products which faciliate and/or improve pigmentation of skin and/or hair, and although good results are obtained by solutions proposed in the prior art, it nevertheless remains that the modulation of pigmentation via the natural route, viz. via the modulation of melanogenesis, is considered the best suited approach.

The technical problem underlying the present invention was to provide means and methods for modulating pigmentation, wherein the modulation of pigmentation is effected by modulating melanogenesis.

Accordingly, this invention relates to a compound having activity in a cell-based tyrosinase assay, obtainable or obtained by (a) culturing *Streptomyces hygroscopicus subsp. hygroscopicus* (DSMZ deposit number DSM 40578); (b) separating the medium containing the compound from the bacterial cells; (c) solid phase extraction of said medium, thereby obtaining a crude extract; (d) performing gel filtration of said crude extract; (e) taking a gel filtration fraction exhibiting activity in said assay; (f) performing HPLC of said fraction exhibiting activity in said assay; (g) taking one or more HPLC fractions exhibiting activity in said assay, thereby obtaining said compound and (h) testing endogenous tyrosinase induction, wherein said assay comprises determining formation of DOPA-quinone from tyrosine or DOPA.

The steps recited above are defined in the general section labelled "Experimental materials and methods" of the Examples enclosed herewith. The method for obtaining the compound(s) of the invention is furthermore exemplified in Example 1. Separating the medium containing the compound(s) of the invention from the bacterial cells of the culture can, for example, be accomplished by centrifugation or filtration. The crude extract is subsequently obtained from the supernatant or the culture filtrate, respectively. The fractions are identified by their activity in the tyrosinase assay detailed further below. Alternatively, and as exemplified in Example 1, the fractions are identified by the retention times as observed when applying the chromatographic protocol as defined in the general section labelled "Experimental materials and methods" of the Examples.

A bacterial isolate *(Streptomyces hygroscopicus subsp. hygroscopicus* (DSMZ deposit number DSM 40578, other deposit numbers: ATCC 27438, CBS 773.72, DSM 41148, IFO 13472, ISP 5578, JCM 4772, NRRL 2387, RIA 1433)) can be used as source for the compounds of the invention. DSM 40578 is a type strain available to the public for at least 30 years from the filing date of the present application (see attached certificate). It is generally used for the taxonomic classification of new microorganisms and for this very reason freely available to the public. In the connection with this use, it is available in the public collection e.g. of DMSZ. The original depositor is E.B. Shirling from the International Streptomyces Project (ISP) at the Ohio Wesleyan University (Delaware, Ohio, 43015 USA).

Alternatively, and as documented in the Examples enclosed herewith, a further bacterial isolate (denoted B333) has been obtained, cultivated and characterized for the identification of bioactive compounds. The present invention also relates to bioactive natural compounds obtainable from isolate B333 from *Streptomyces sp.* It was found that the methanol extracts of isolate B333 contain the compounds defined above, which, in accordance with this invention, surprisingly have various biotechnological, cosmetic as well as pharmaceutical applications.

The bacterial isolates B333 and any *Streptomyces hygroscopicus subsp. hygroscopicus* which can be used without undue burden for the isolation of the compounds of the invention can reliably be obtained by steps per se known in the art and exemplified in Example 1. Generally, a soil sample, preferably from a geranium flower bed, is dried and resuspended in water. The mycelium and the spores are separated from the associated organic particles by methods well known in the art. A dilution series of the suspension is prepared and plated on agar plates containing a suitable medium. Colonies of *Streptomyces* are readily recognized by the skilled person. Further details may be taken or derived from Example 1. Subsequently, and as recited in the main embodiment, a crude extract is obtained from cultures of the microorganism obtained as described above, purified, and tested for its activity in an assay of the invention further detailed below.

These compounds and their derivatives and modifications disclosed herein have melanogenesis-modulating activities in mammalian cells. Prior art compounds with similar spectroscopic data (for a comparison see Example 2) have been described to exhibit antimicrobial, in particular, antifungal activities (Ivanova et al. (2000)).

The class of actinobacteria (formerly denoted actinomycetes) has an exceptional position amongst the kingdom of bacteria and still remains a challenging target for exploitation in order to find new organisms with interesting biosynthetic profiles. These profiles are generally considered to be complex screening materials, requiring extensive and costly chemical fractionation of microbial extracts. However, these former approaches are now being superseded by innovative technologies and sophisticated, robust assay systems allowing the identification of compounds with new biological effects which in turn may be exploited for cosmetic of pharmaceutical applications.

To deal with a complex screening material a strain library of approximately 600 isolates (basically actinobcteria) were analysed for potential redundancy according to data from previous screenings with this library. To seperate out potential redundancy of the actinobacteria in terms of morphologic, phylogenetic and chemical diversity exactly this data set of previous screening experiences was taken into account. In a first step the 600 isolates were compared with regard to their morphologic similarities (growth, colour, shape and mycelium). Further on the phylogenetic dataset of the remaining strains was assessed by 16S rDNA genotyping using ARB software and database (Strunk et al. (1997)). Additional selection of organisms was accomplished by metabolic finger-printing using HPLC-DAD data (ranging from 200 to 650 nm); sorting out those isolates which show similar HPLC profiles. As a consequence of this procedure, which can be termed as rational bioprospecting, a value-added-library of roughly 50 isolates (one tenth of the original library size) of secondary metabolites was generated, leading to a drastic reduction of screening efforts. Despite of the high complexity of the task (crude extracts combined with cellular systems), this screening set-up has surprisingly been shown to exhibit selectivity and specificity, as primary hits with melanin biosynthesis modulation activities were found within those preselected isolates.

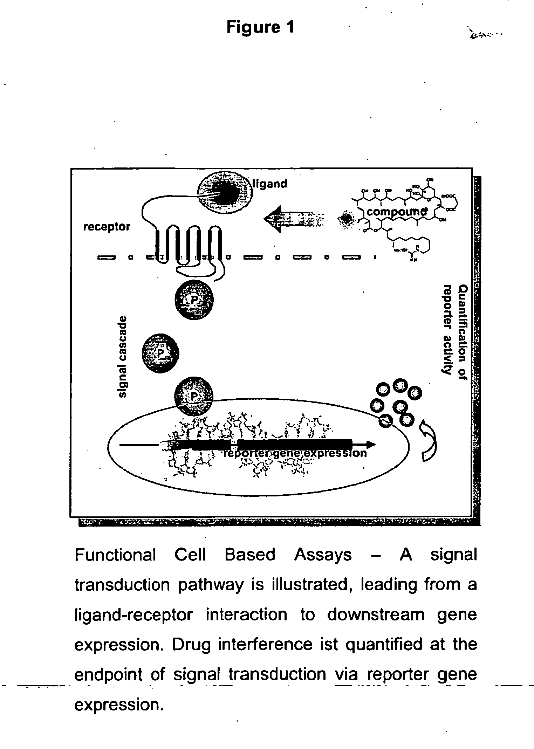

The majority of pharmacologically relevant molecules are involved in cellular signal transduction pathways. For these targets, functional assays based on surrogate cells have been used with either cell-inherent physiological pathways or with recombinant, quantifiable, non-radioactive reporter system readouts (see Fig. 1). Considering melanin biosynthesis, the melanoma cell line S91 (ATCC-No. CCL 53.1)-based pigmentation assay as well as a recombinant cell-based reporter gene assay were specifically combined and tested with extracts of preselected actinomycetes and other microbes to identify new compounds or'bio-ceuticals'. S91 cells, which express tyrosinase, the rate-limiting enzyme in pigmentation processes, were screened for compounds with potential effects on pigmentation using aqueous extracts of selected bacteria. S91 melanogenesis stimulation leads to a visually apparent increase in cellular melanin concentrations. Although a change in color of the cells could be observed, it was not sufficient for quantification. Therefore, intrinsic tyrosinase activities were measured using a colorimetric read out (see Examples 3 and 5). Experiments to evaluate cytotoxic activities of the extracts tested were performed in parallel and taken into consideration prior to the identification of primary hits. In terms of the cytotoxic data of the 50 isolates used for the here described screening approach, it was possible to adjust the extracts to sub-cytotoxic concentrations within the tyrosinase or reporter gene assays.

Based on the assumption that a compound modulates melanogenesis while interfering with the cAMP signaling pathway, inducible by the melanocortin 1 receptor, a cAMP response element (CRE) binding cell based assay was used to analyse such bioactive compounds (see Example 4). Subsequently, the melanocortin receptor's cell-inherent signal transduction pathway is coupled to a CRE response element controlled reporter gene, leading to a quantifiable, non-radioactive readout. In this kind of assays the increase of the levels of second messenger cAMP ― generated by the binding of alpha-MSH to MC1R ― is monitored by a luciferase reporter gene (see also Fig. 1). With this assay it was shown that the aqueous extracts, identified as primary hit extracts which induce S91 endogeneous tyrosinase, were also able to induce CRE-dependent luciferase activity.
For this reason the extract of the bacterial isolate mentioned above ― which is probably closely related to *Streptomyces hygroscopicus* according to its chemical finger-print and 16S rDNA data ― was selected for further fractionation and purification by gel chromatography and preparative HPLC. From the physico-chemical properties (see Example 2) it can be inferred with high probability that the here described bioactivities are based on two structurally related compounds of the niphimycin family.
The above mentioned assays, also subject of the present invention, are further detailed below.

In a further embodiment, this invention relates to a pharmaceutical or cosmetic composition comprising the compound defined above. Also described is a method of cosmetic treatment comprising the administration of the compound defined above to a subject in need of or desiring such treatment. Furthermore, the compound of the present invention can be used for the preparation of a cosmetic composition, or of a pharmaceutical composition, the latter being for the treatment of pigmentation disorders.

Also described herein is a method of cosmetic treatment comprising the administration of a compound represented by the general formula (I) 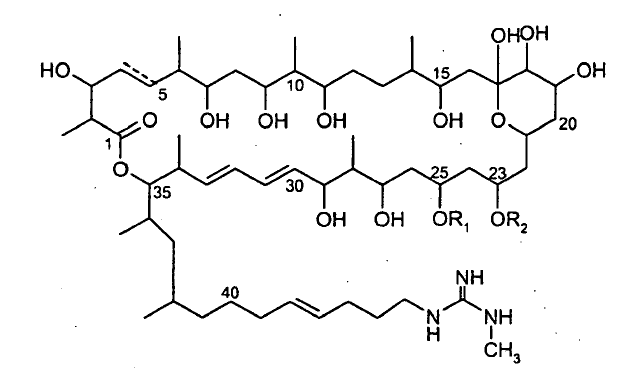 wherein the bond between C₄ and C₅ is either a single bond or a double bond, to a subject in need of or desiring such treatment. In a further embodiment, this invention relates to the use of a compound represented by the general formula (I), wherein the bond between C₄ and C₅ is either a single bond or a double bond, for the preparation of a cosmetic composition.

In a preferred embodiment of the method or the use of the invention, the bond between C₄ and C₅ is a single bond. According to the use of the invention, R₁ is H and R₂ is ―COCH₂COOH (malonyl), or another preferred embodiment, R₁ is ―COCH₂COOH (malonyl) and R₂ is H.

So far bioactivities of macrolides of the niphimycin type have only been discussed in terms of being antifungal and active again Gram-positive bacteria (Ivanova V. & Gushterova A., 1997) or as inhibitors of the type-I Interleukin-I Receptor (Stefanelli S. et al, 1997).

Also described herein are methods using variant compounds and uses of variant compounds. Said variant compounds are variant compounds of the compounds of general formula (I), having the same molecular formula as the compounds of formula (I), wherein, as valence and stability permits, the positions of the methyl and hydroxy groups are variable with regard to regio- and stereochemistry. In other words, one hydrogen of any methylene group may be replaced with a methyl group, provided that a methyl group elsewhere present in formula (I) is concomitantly removed. Similarly, one hydrogen of any methylene group may be replaced with a hydroxy group, provided that a hydroxy group elsewhere present in formula (I) is concomitantly removed. Also, a methyl and a hydroxy group present in formula (I) may be interchanged. Any combination of the above described replacement steps is envisaged as is their repeated application.

In a preferred embodiment, or cosmetic compositions of the invention are to be applied to skin, hair and/or eyes of a human subject or of an animal.

In a further preferred embodiment of the use of the present invention, the application of the cosmetic composition modulates the pigmentation of the body part to which said cosmetic composition is applied to.

More preferred are uses of the present invention, wherein the application of the cosmetic composition brings about pigmentation of the body part to which said cosmetic composition is applied to. Yet more preferred are uses, wherein said composition is to be administered to the skin, and a tanned skin is brought about by the administration and/or sun protection is provided thereby.

Also described are uses, wherein said composition is to be administered to the skin and a reduction of pigmentation, a whitened skin, or a removal of hyperpigmentation is achieved thereby. This effect can be achieved by selecting a suitable specific compound of general formula (I) and/or selecting certain conditions for administration. Compounds of general formula (I) suitable for a reduction of pigmentation can be identified with the assays disclosed below. Preferred embodiments of the uses described above are uses, wherein said hyperpigmentation or pigmentation to be reduced is selected from age spots and freckles.

In a further embodiment of the present invention, the compound represented by the general formula (I) is used for the preparation of a pharmaceutical composition for the treatment of pigmentation disorders.

Pigmentation disorders according to the present invention are preferably selected from vitiligo and albinism, such as oculocutaneous albinism type 1, 2 or 3.

Also described herein are specific tests or assays which are useful in further screenings programmes of the compounds described herein. These tests are illustrated in the appended examples and comprise tests for cytotoxicity, inter alia, for interference with the cAMP response signaling pathway of eukaryotic cells, or for activity in melanin biosynthesis.

Accordingly, described is a method for identifying modulators of melanogenesis comprising: (a) determining the cellular melanin concentration of a cell capable of producing melanin; (b) incubating said cell with a test compound such that the compound binds to the surface of the cell or is taken up by the cell; (c) determining the cellular melanin concentration; (d) comparing the cellular melanin concentrations determined in steps (a) and (c); and (e) identifying compounds that alter the cellular melanin concentration, thereby identifying modulators of melanogenesis.

Also described is a method for identifying modulators of melanogenesis comprising: (a) incubating a cell expressing tyrosinase with a test compound such that the compound binds to the surface of the cell or is taken up by the cell; (a') incubating a cell expressing tyrosinase with a reference compound such that the compound binds to the surface of the cell or is taken up by the cell, or with no compound; (b) adding a tyrosinase substrate to the cell incubated in step (a); (b') adding a tyrosinase substrate to the cell incubated in step (a'); (c) comparing the amount of product formed from said substrate added in steps (b) and (b'); and (d) identifying compounds that alter the amount of product formed, thereby identifying modulators of melanogenesis, wherein step (a') may be performed prior to step (a), during step (a) or (b), or prior to step (c) and wherein step (b') is performed after step (a').

The term "tyrosinase" refers to any enzyme exhibiting tyrosinase activity. The tyrosinase activity may be defined as the enzymatic activity which catalyzes the oxidation of tyrosine resulting in the formation of the melanin precursor dopaquinone. Enzymes designated "tyrosinase" herein may exhibit also other enzymatic activities in addition to the above described activity. The method described above is also referred to as "tyrosinase assay", "tyrosinase activity assay" or "cell-based tyrosinase assay" herein and is exemplified in Example 3.

The product recited above may be detectably labelled and/or absorbs light.

Said substrate may be DOPA, and said product may be DOPA-quinone, and said light may be visible light with a wavelength of about 475 nm.

Moreover, described is a method for identifying modulators of melanogenesis is provided, comprising: (a) incubating a cell capable of producing melanin and comprising a reporter gene operably linked to an expression control element with a test compound such that the compound binds to the surface of the cell or is taken up by the cell; (a') incubating a cell capable of producing melanin and comprising a reporter gene operably linked to an expression control element with a reference compound such that the compound binds to the surface of the cell or is taken up by the cell, or with no compound; (b) determining the activity of the reporter gene in the cell incubated in step (a); (b') determining the activity of the reporter gene in the cell incubated in step (a'); (c) comparing the activities determined in steps (b) and (b'); and (d) identifying compounds that alter the activity of the reporter gene, thereby identifying modulators of melanogenesis, wherein step (a') may be performed prior to step (a), during step (a) or (b), or prior to step (c) and wherein step (b') is performed after step (a').

In the described methods, compounds may be identified that increase the cellular melanin concentration, or increase the amount of product formed, or increase the activity of the reporter gene, thereby identifying activators of melanogenesis.

Also described is that compounds are identified that reduce the cellular melanin concentration, or reduce the amount of product formed, or reduce the activity of the reporter gene, thereby identifying inhibitors of melanogenesis.

The cell used in said methods may be melanoma cell line S91 (ATCC No. CCL 53.1).

In the methods using a reporter gene operably linked to an expression control element, the reporter gene may be a luciferase gene, and the expression control element is SV40 promoter.

The reference compound recited above may be alpha-melanocortin stimulating hormone (alpha-MSH), adrenocorticotropic hormone (ACTH), beta-MSH or gamma-MSH.

The present invention also provides a compound represented by formula (II) 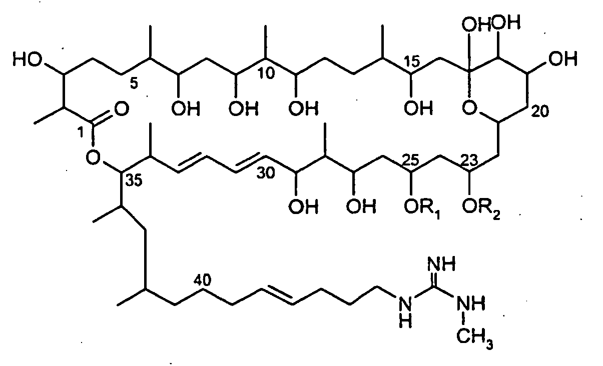 wherein R₁ = -COCH₂COOH (malonyl) and R₂ = H.

Also described is a compound represented by formula (III) 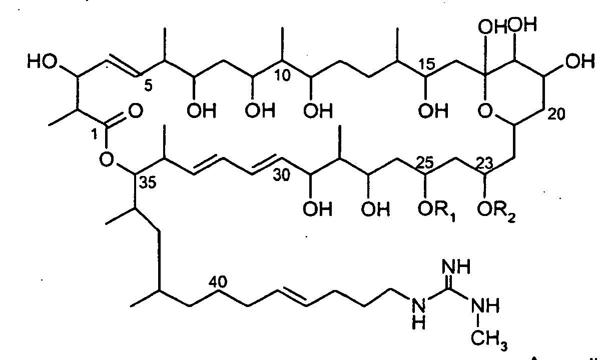 wherein R₁ = -COCH₂COOH and R₂ = H, is provided. According to the invention, formulation of the compounds of formula (II) and (III) into a pharmaceutical or cosmetic composition is envisaged.

According to the present invention the chemical formulae given include all isomers, stereoisomers and diastereomers. If the compounds are chiral, the present invention is intended to cover racemates, mixtures of the stereoisomers in varying ratios as well as the chiral compounds as such.

The compounds recited herein may form salts which are also within the scope of this invention. Reference to a compound of the invention is understood to include reference to salts thereof, unless otherwise indicated. The term "salt(s)", as employed herein, denotes acidic and/or basic salts formed with inorganic and/or organic acids and bases. In addition, when a compound of the invention contains both a basic moiety and an acidic moiety, zwitterions ("inner salts") may be formed and are included within the term "salt(s)" as used herein. Pharmaceutically acceptable (i.e., non-toxic, physiologically acceptable) salts are preferred, although other salts are also useful, e.g., in isolation or purification steps which may be employed during preparation. Salts of the compounds of the invention may be formed, for example, by reacting a compound of the invention with an amount of acid or base, such as an equivalent amount, in a medium such as one in which the salt precipitates or in an aqueous medium followed by lyophilization.

The compounds recited herein which contain a basic moiety may form salts with a variety of organic and inorganic acids. Exemplary acid addition salts include acetates, adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, 2-hydroxyethanesulfonates, lactates, maleates, methanesulfonates, 2-naphtalenesulfonates, nicotinates, nitrates, oxalates, pectinates, persulfates, 3-phenylpropionates, phosphates, picrates, pivalates, propionates, salicylates, succinates, sulfates, sulfonates, tartrates, thiocyanates, toluenesulfonates, such as tosylates, undecanoates, and the like.

The compounds recited herein which contain an acidic moiety may form salts with a variety of organic and inorganic bases. Exemplary basic salts include ammonium salts, alkali metal salts such as sodium, lithium, and potassium salts, alkaline earth metal salts such as calcium and magnesium slats, salts with organic bases (for example, organic amines) such as benzathines, dicyclohexylamines, N-methyl-D-glucamines, N-methyl-D-glucamides, t-butyl amines, and salts with amino acids such as arginine, lysine and the like.

Prodrugs and solvates of the compounds recited herein are also contemplated herein. The term "prodrug", as employed herein, denotes a compound which, upon administration to a subject, undergoes chemical conversion by metabolic or chemical processes to yield a compound of the invention, or a salt and/or solvate thereof. Solvates of the compounds of the present invention are preferably hydrates.

To the extent that compounds recited herein and salts thereof may exist in their tautomeric form, all such tautomeric forms are contemplated herein as part of the present invention.

All stereoisomers of the present compounds, such as those which may exist due to asymmetric carbons on the various substituents, including enantiomeric forms (which may exist even in the absence of asymmetric carbons) and diastereomeric forms, are contemplated within the scope of this invention. Individual stereoisomers of the compounds of the invention may, for example, be substantially free of other isomers, or may be admixed, for example, as racemates or with all other, or other selected, stereoisomers.

Where desired, the compounds of the invention may be used in combination with one or more other types of therapeutic agents which may be administered of the same type (e.g. orally) in the same dosage form (e.g. orally), in a separate dosage form or by a different application form (e.g. by injection).

It is preferred that the pharmaceutical composition of the invention comprise, optionally, a pharmaceutically acceptable carrier and/or diluent.

Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by oral, rectal, intravenous, intraperitoneal, subcutaneous, intramuscular, topical, intradermal, intranasal or intrabronchial administration. It is particularly preferred that said administration is carried out by injection and/or delivery, e.g., to a site in an artery or directly into tissue. The compositions of the invention may also be administered directly to the target site, e.g., by biolistic delivery to an external or internal target site. The dosage regimen will be determined by the attending physician and clinical factors. As is well known in the medical arts, dosages for any one patient depend upon many factors, including the severity of the condition, the patient's size, body surface area, age, the particular compound to be administered, sex, time and route of administration, general health, and other drugs being administered concurrently. Pharmaceutically active matter may be present in amounts from 1 ng to 10 mg/kg body weight per dose; however, doses below or above this exemplary range are envisioned, especially considering the aforementioned factors. If the regimen is a continuous infusion, it should also be in the range of 1 µg to 10 mg units per kilogram of body weight per minute.

The Figures show:
**Figure 1****:** Functional Cell Based Assays ― A signal transduction pathway is illustrated, leading from a ligand-receptor interaction to downstream gene expression. Drug interference ist quantified at the endpoint of signal transduction via reporter gene expression.

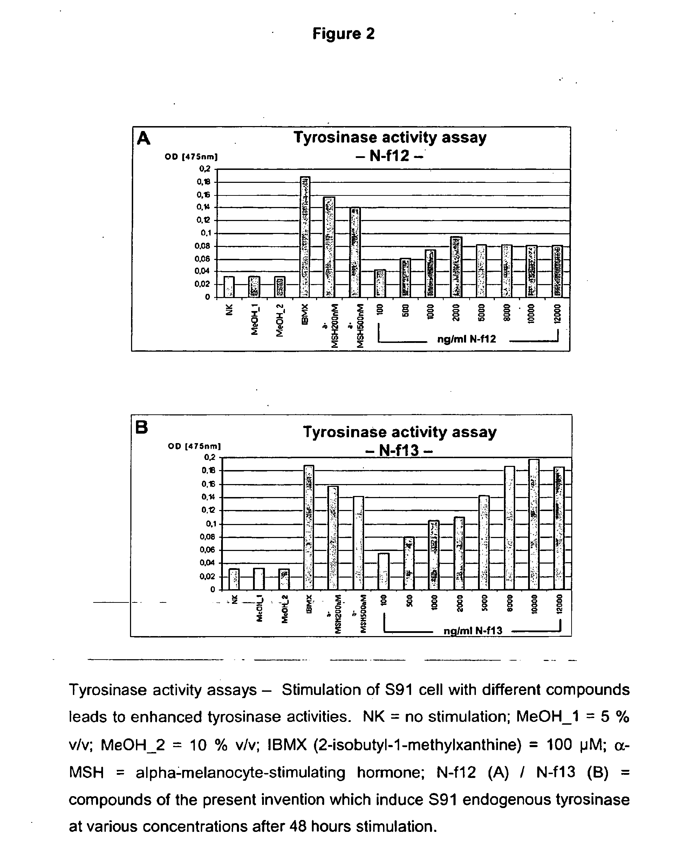

**Figure 2****:** Tyrosinase activity assays ― Stimulation of S91 cell with different compounds leads to enhanced tyrosinase activities. NK = no stimulation; MeOH_1 = 5 % v/v; MeOH_2 = 10 % v/v; IBMX (2-isobutyl-1-methylxanthine) = 100 µM; α-MSH = alpha-melanocyte-stimulating hormone; N-f12 (A) / N-f13 (B) = compounds of the present invention which induce S91 endogenous tyrosinase at various concentrations after 48 hours stimulation.

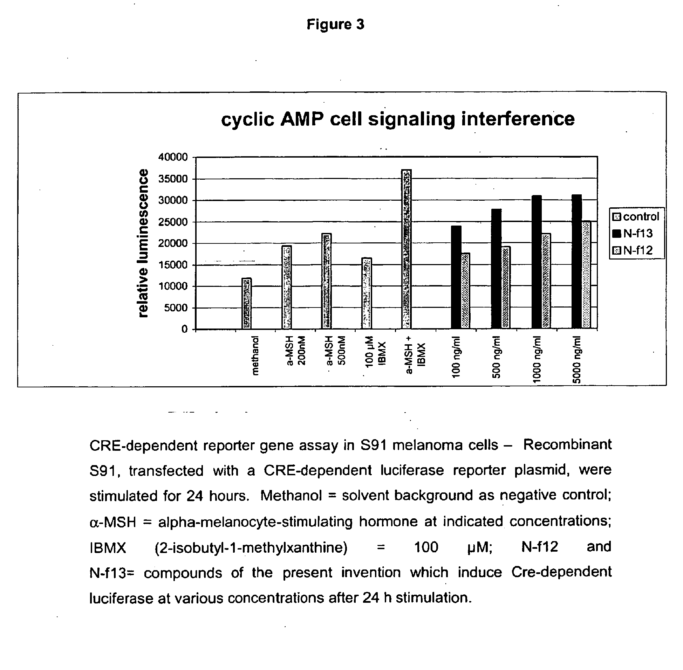

**Figure 3****:** CRE-dependent reporter gene assay in S91 melanoma cells ― Recombinant S91, transfected with a CRE-dependent luciferase reporter plasmid, were stimulated for 24 hours. Methanol = solvent background as negative control; α-MSH = alpha-melanocyte-stimulating hormone at indicated concentrations; IBMX (2-isobutyl-1-methylxanthine) = 100 µM; N-f12 and N-f13= compounds of the present invention which induce Cre-dependent luciferase at various concentrations after 24 h stimulation.

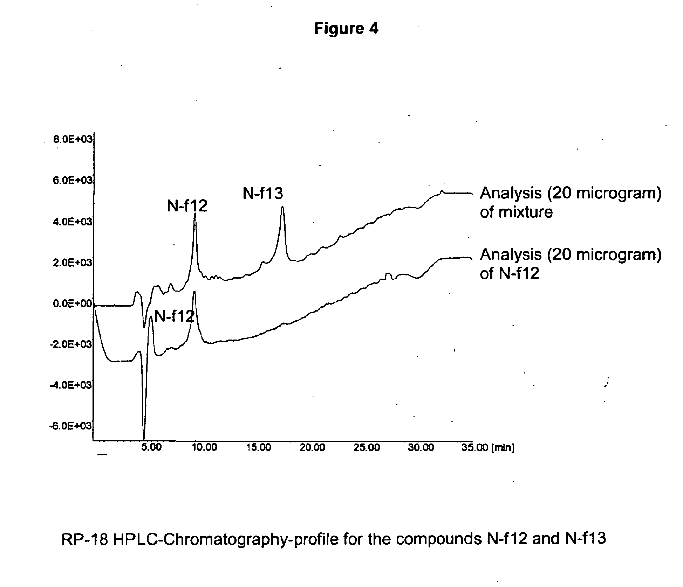

**Figure 4****:** RP18 HPLC - Chromatography profile

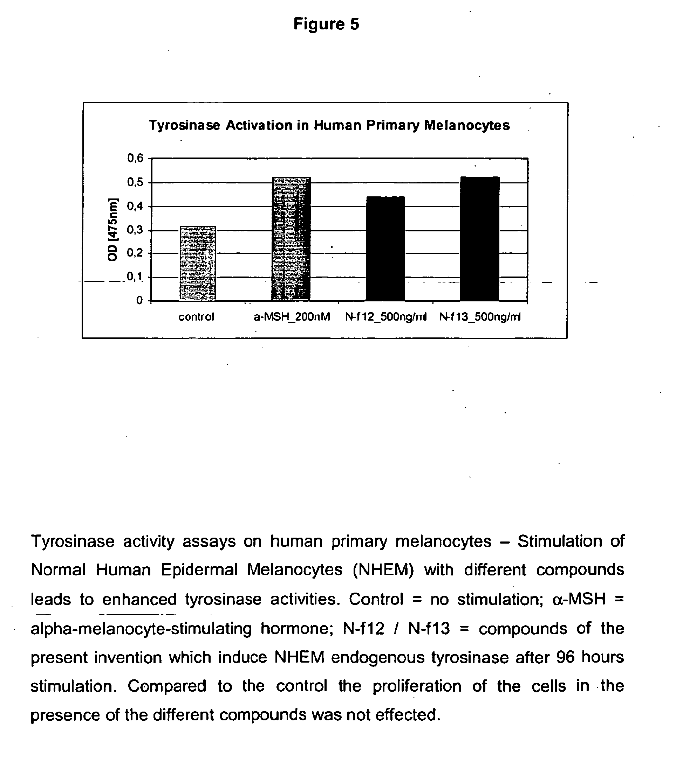

**Figure 5****:** Tyrosinase activity assays on human primary melanocytes ― Stimulation of Normal Human Epidermal Melanocytes (NHEM) with different compounds leads to enhanced tyrosinase activities. Control = no stimulation; α-MSH = alpha-melanocyte-stimulating hormone; N-f12 / N-f13 = compounds of the present invention which induce NHEM endogenous tyrosinase after 96 hours stimulation. Compared to the control the proliferation of the cells in the presence of the different compounds was not effected.

The following examples illustrate the invention but should not be construed as being limiting.

### Experimental materials and methods

**MS spectra: ESI-MS:** Finnigan LQC, Bruker FT-ICR.

**UV spectra:** Kontron Uvikon 860, Varian Modell Cary 3E; Methanol/HCl bzw. Methanol/NaOH:

**¹H-NMR spectra:** Varian Inova 600 (600 MHz). Chemical shifts in δ-values (ppm) relative to the respective solvent as internal standard. All ¹H-NMR spectra were interpretated as of first order.

**¹³C-NMR** spectra: Varian Inova 600 (150.8 MHz). Chemical shifts in δ-values (ppm) relative to the respective solvent as internal standard.

**Solvents:** All solvents for chromatrography were destillated. For all other applications HPLC-grade solvents were used.

**TLC analysis:** Nano-TLC plates RP-8 F254 (Merck): 0.25 mm.

**Staining reagents:** Merck, "Anfärbereagenzien für die Dünnschichtchromatographie". All plates were heated to 100 C after staining. Anisaldehyde (*No. 21*): 1.0mL anisaldehyde in a solution of 85mL methanol, 10mL acetic acid and 5mL H₂SO₄ conc.

### Column and gel chromatography:

Gel filtration: Sephadex LH-20 (Amersham Biosciences); gravity column; solvent: methanol.

Reversed phase medium pressure liquid chromatography (RP-MPLC): RP-18 MPLC-silica gel (Merck); solvent: MeOH:H₂O, 9:1; Lobar® column, 30 x 2.5 cm, 1 bar.
Preparative HPLC: Nucleosil 100 C18; solvent: acetonitrile/1%H₂O/0.1%TFA in H₂O/0.1%TFA

**Solid phase extraction:** adsorbent resin: Amberlite® XAD-2 (Serva); washing with H₂O dest.; elution with 100% methanol. Subsequently, methanol is evaporated in vacuo and the extract dried by lyophilization. The dry extract is then taken up in methanol.

**HPLC:** pump: Kontron 322 System; autosampler: Kontron 360; detector Kontron Diode Array Detector 440; mix chamber: Kontron HPLC 360; data system: Kontron Kroma-System 2000 version 1.60; pre-column: Beckmann Ultrasphere Gold ODS, 4.6 x 45 mm, 5 µm, RP-18; column: Grom Ultrasphere 100 RP-18 endc. 4, 100 x 2 mm, flow rate: 1.0 mL/min, gradient 100 % H₂O to 100 % methanol.

**Cultivation of bacterial isolates:** Batch submerse cultivation in aqeous Starch Asparagine Medium/l (20.00 g potato starch, 1.50 g L-asparagine, 1.00g KCI, 1.00g CaCO₃, 20.00 mL trace element solution H, 1.00 g D-(+)-glucose (anhydrous). Adjusted to pH=8.0 with 10N NaOH, autoclaved 20 min at 121°C).

**Media:**
- GHPF Medium/I: 10.00 g D-(+)-glucose (anhydrous), 5.00 g peptone from casein, 5.00 g meat extract, 5.00 g yeast extract, 0.74 g CaCl₂ x H₂O. Adjusted to pH=7.2.
- Starch Asparagine Medium/I: 20.00 g potato starch, 1.50 g L-asparagine, 1.00g KCl, 1.00 g CaCO₃, 20.00 mL trace element solution H, 1.00 g D-(+)-glucose (anhydrous). Adjusted to pH=8.0 with 10N NaOH, autoclaved 20 min at 121°C.
- GYM Medium/l: 4.0g Glucose, 4g yeast extract, 10.0g malt extract, 2.0g CaCO₃, 12.0g Agar; adjusted to pH=7.2 with KOH before adding agar.

### Example 1: Isolation and fermentation of the bacterial isolate B333 (Streptomyces sp) and compound isolation

The bacterial isolate B333 was isolated from a soil sample, which was taken from a geranium flower bed. The isolation procedure encompassed standard microbiological methods described for the isolation of organisms of the bacterial family streptomycetaceae such as dilution plate technique (Korn-Wendisch and Kutzner, 1991). Briefly, the soil sample (10g) was dried (40-45°C) for 16h prior to resuspension in 100mL H₂O. To separate the vegetative mycelium and the spores from associated organic particles glass beads were added to the suspension and agitated on a lab shaker. Agar plates containing GYM media were surface-inoculated (100µL) with dilution series of the suspension from 10⁻² up to 10⁻⁶ and incubated at 28°C for several days. To suppress growth of other sporulating organisms such as fungi, the antibiotic cycloheximid was added to the media (50µg/mL). Colonies of Streptomyces are readily recognized by their macroscopic and microscopic features and can be easily purified by transferring colonies to fresh media plates. After several rounds of isolation/purification plating the isolate B333 appears with a light grey arial mycelium, which turns darker up to black after several day at 28°C.
The compounds of the present invention can be obtained from the culture broths of isolate B333 grown according to known biotechnological procedures in a batch submerse cultivation process with standard nutrition broths. After sufficient growth of the microorganism, the cultivation is terminated by separation of the cells. Briefly, the biomass and culture broth were separated using centrifugation or filtration. The compounds are then isolated from the culture filtrate. The initial work-up procedure involves adsorption on solid phase resin (e.g. Amberlite® XAD-2) and elution of the bound compounds with methanol. After evaporation of the organic solvent, the compounds are separated from unwanted material by standard chromatographic procedures (e.g. on silica gel using chloroform/methanol gradients).
The metabolite pattern and the yields of the different compounds is highly dependent on the composition of the culture medium. For the production of the niphimycin N-f12 and N-f13 in a 5 L-fermentor, we cultivated the baterial isolate B333 in a medium containing 2% (w/v) starch and 0.15% (w/v) asparagine. For inoculation 10% of preculture in GHPF medium which was cultivated for 48 hours at 30°C was used. The production of the compounds was monitored by thin layer chromatography (TLC) and HPLC. After incubation for 120 hours at 30°C the culture was harvested.
For the isolation of the metabolites the mycelium of isolate B333 was separated by centrifugation. The separated bacterial cells of the 5L fermentation were extracted with 3 x 250mL of acetone. The organic solvent was evaporated in vacuo and the extract dried by lyophilisation to yield 0.68g of crude extract. This extract contains a certain amount of the dihydroniphimycin derivatives which were not further quantified and not further isolated, since they yielded poor activity in the tyrosinase assay.
For solid phase extraction, 2.5L of the supernatant were applied to 1 L XAD-2 resin which was washed with 2.5L H₂O dest and treated with 1 L methanol (100%). The organic solvent was evaporated in vacuo and the extract dried by lyophilisation. This procedure was carried out twice to yield 1.6g of crude extract. 900mg of of the crude extract were methanol soluble and applied to gel filtration chromatography using Sephadex LH-20 (gravity column: 100x2.5 cm, MeOH as solvent) to yield at first 28.4mg of the enriched dihydroniphimyin-derivative fraction. This fraction showed significant activity in the tyrosinase assay.
[Other fractions: 2: 122.2mg, 3: 147.4mg, 4: 259.6mg, 5: 58.3mg, 6: 24.2mg, 7: 9.9mg].

RP-18 MPLC-silica gel chromatography (MeOH:H₂O, 9:1; MERCK, Lobar column, 30 x 2.5cm, 1 bar) with the 28.4mg of the enriched fraction afforded 4.5mg of pure product N-f12 as 2^{nd} fraction and 7.2 mg of the pure derivative N-f13 as 4^{th} fraction. Both fractions show activity in the tyrosinase assay of the invention. The solvent was removed by evaporation in vacuo and by lyophylisation. The activity of the pure compounds was tested in the tyrosinase assays as described in Example 3 and 4. The purity was determined by HPLC-DAD-analysis, TLC analysis and mass spectrometry.
[Other fractions: 1: 1.1 mg and 3: 5.0mg of a mixture of N-f12 and N-f13]
Alternatively, preparative HPLC-chromatography (Nucleosil, 100 C18, flow-rate: 2ml/min) also yields pure N-f12 (Rₜ=9.8 min) and N-f13 (Rₜ= 17.5 min). Again, both fractions show activity in the tyrosinase assay of the invention. For this a gradient program was used starting with 45% increasing to 65% acetonitrile/1%H₂O/0.1%TFA in H₂O/0.1%TFA in 35 min. 80µL of a 1 mg/mL solution of the mixture of N-f12 and N-f13 in isopropanol was applied for preparative purposes in each single run. The solvent is removed by evaporation in vacuo under repeated addition of methanol to azeotropically remove traces of acid and finally by lyophylisation.
See examples of the analytical tests. 20 µL of 1 mg/mL dihydroniphimycin in MeOH were injected. In Figure 4 upper chromatogram: mixture of N-f12 and N-f13. Lower chromatogram: pure N-f12.
The cultivation of isolate B333 in a medium with 2% w/v oatmeal and trace element solution yielded natural products known in the literature: 15.2 mg/l of hygromycin A, 1.0 mg/L 1-desoxy-2-pentulose, 4.8 mg/L geldanamycin, 0.5 mg/L thymidine and other substances.

### Example 2: Structural assignment of the bioactive compounds

The chemical formulae of the compounds was determined by HR-ESI-MS by FT-ICR-technique to be C₅₉H₁₀₅N₃O₈ for both of the compounds. The UV spectrum showed absorption maxima at 231 nm. The ¹H- and ¹³C-NMR data indicated the presence of a macrolactone ring. These data were compared with the literature and databases to proof the isolated compounds N-f12 and N-f13 to be two different members of the niphimycin family. When treated with anisaldehyde stain the metabolites N-f12 and N-f13 show intense blue and violet coloration, respectively. Different R_{f}- and Rₜ- values confirm the existence of two different substances, which are both acid-sensitive white powders, but stable for months at -20°C in methanol.
Both compounds N-f12 and N-f13 show spectroscopic properties very similar to those of dihydro derivatives of the parent compound niphimycin Iα (C₅₉H₁₀₃N₃O₈). The spectroscopic data are summarized in Tables 1 and 2 shown below.

**Table 1: Comparison of the analytical data of the dihydroniphimycin derivatives N- f12 and N-f13:**

| | | | | | | | |
|---|---|---|---|---|---|---|---|
| 1. ¹H- und ¹³C-NMR spectroscopical data in comparison with the known 4,5- dihydroniphimycin and malonyl niphimycin. * - assignments not confirmed "diff" ― distinct differences of spectroscopical data "*id*" ― very similar or identical data of characteric regions chemical shifts in ppm | | | | | | | |

| **N-f12** | **N-f12** | **N-f13** | **N-f13** | | **4,5-Dihydro- niphimycin** *J. Antibiot.* **2000,** *53*, 627-632. | **4,5-Dihydro- niphimycin** | **Malonyl- niphimycin** *J. Antibiot.* **1997,** 50, 965-969. |
|---|---|---|---|---|---|---|---|
| **¹³C- NMR- data*** (150.82 MHz, cd₃od+2 Tr. cdcl₃) | **¹H-NMR- data*** (500 MHz, cd₃od+2 Tr. cdcl₃) | **¹³C-NMR- data*** (150.82 MHz, cd₃od+2 Tr. cdcl₃) | **¹H-NMR- data*** (cd₃od, 600 MHz) | **C- Atom** | **¹³C-NMR-data** (100.62 MHz, cd₃od) | **¹H-NMR-data** (400 MHz) | **¹³C-NMR- data** (100.62 MHz, cd₃od) |
| *176.9 id* | - | *176.9 id* | - | ***1*** | *176,80 id* | | *176,8 id* |
| 46.9 | 1H, 2.41 | 46.6 | 1H, 2.40 | **2** | 46,89 | 1H, 2,41 | 48,0 |
| 72.9 | 1H, 3.71 | 72.8 | 1H, 3.71 | **3** | 72,96 | 1H, 3,72 | 65,5 |
| **37.2 diff** | 1.20-1.90 | **37.6 diff** | 1.20-1.90 | **4** | **37,40 diff** | 2H, 1,50 | **132,5 diff** |
| 28.3 | 1.20-1.90 | 28.3 | 1.20-1.90 | **5** | 29,38 | 2H, 1,31/1,50 | **136,5 diff** |
| 40.2 | 1.20-1.90 | 40.5 | 1.20-1.90 | **6** | 39,91 | 1H, 1,55 | 43,4 |
| 77.3 | 1H, 3.63 | 77.3 | 1H, 3.64 | **7** | 77,27 | 1H, 3,65 | 76,0 |
| **33.9 diff** | 1.20-1.90 | **37.2 diff** | 1.20-1.90 | **8** | **37,37 diff** | 2H, 1,50/1,80 | 39,3 |
| 76.3 | 3.61-3.92 | 76.4 | 3.78-3.93 | **9** | 76,34 | 1H, 3,82 | 75,1 |
| 44.3 | 1.20-1.90 | 44.6 | 1.20-1.90 | **10** | 44,48 | 1H, 1,54 | 45,3 |
| 72.0 | 3.61-3.92 | 72.0 | 3.78-3.93 | **11** | 72,28 | 1H, 3,85 | 72,4 |
| 33.4 | 1.20-1.90 | 33.9 | 1.20-1.90 | **12** | 33,96 | 2H, 1,39/1,61 | 33,5 |
| **39.9 diff** | 1.20-1.90 | **39.8 diff** | 1.20-1.90 | **13** | **37,72 diff** | 2H, 1,07/1,26 | 37,6 |
| 33.0 | 1.20-1.90 | 33.5 | 1.20-1.90 | **14** | 30,59 | 1H, 1,62 | 30,6 |
| 66.0 | 3.61-3.92 | 65.3 | 3.78-3.93 | **15** | 65,53 | 1H, 3,84 | 65,8 |
| 41.9 | 1.20-1.90 | 41.9 | 1.20-1.90 | **16** | 41,90 | 2H, 1,66/1,80 | 42,0 |
| *99.7 id* | *-* | *99.6 id* | *-* | ***17*** | *99,68 id* | *-* | *100,0 id* |
| 77.2 | 3.61-3.92 | 76.8 | 3.78-3.93 | **18** | 77,07 | 1H, 3,35 | 74,2 |
| 69.6 | 3.61-3.92 | 69.5 | 3.78-3.93 | **19** | 69,65 | 1H, 3,87 | 74,2 |
| 41.1 | 1.20-1.90 | 41.2 | 1.20-1.90 | **20** | 41,22 | 2H, 1,30/1,90 | 38,2 |
| 65.4 | 1H, 4.01 | 65.1 | 1H, 4.07 | **21** | 65,40 | 1H, 4,07 | 76,0 |
| 41.5 | 1.20-1.90 | 41.8 | 1.20-1.90 | **22** | 41,48 | 2H, 1,66/1,80 | 41,7 |
| 71.0 | 1H, 5.25 | 70.4 | 1H, 5.25 | **23** | 70,66 | 1H, 5,25 | 71,6 |
| * | 1.20-1.90 | * | 1.20-1.90 | **24** | 41,48 | 2H, 1,66/1,80 | 41,7 |
| 72.3 | 3.61-3.92 | 72.3 | 3.78-3.93 | **25** | 72,48 | 1H, 3,85 | 72,4 |
| 43.7 | 1.20-1.90 | 43.6 | 1.20-1.90 | **26** | 43,66 | 2H, 1,50/1,64 | 43,1 |
| 68.3 | 1H, 4.16 | 68.5 | 1H, 4.16 | **27** | 68,86 | 1H, 4,16 | 69,3 |
| 44.4 | 1.20-1.90 | 44.7 | 1.20-1.90 | **28** | 45,24 | 1H, 1,57 | 44,5 |
| 75.2 | 1H, 3.98 | 75.8 | 1H, 4.03 | **29** | 75,90 | 1H, 4,03 | 75,6 |
| *134.8 id* | *1H, 5.62* | *135.4 id* | *1H, 5.63* | ***30*** | *135,36 id* | *1H, 5,63* | *135,2 id* |
| *132.3 id* | *1H, 6.15* | *132.2 id* | *1H, 6.17* | ***31*** | *132,15 id* | *1H, 6,18* | *132,0 id* |
| *131.8 id* | *1H, 6.06* | *131.9 id* | *1H, 6.05* | ***32*** | *131,90 id* | *1H, 6,05* | *131,9 id* |
| *136.9 id* | *1H, 5.53* | *137.0 id* | *1H, 5.50* | ***33*** | *136,97 id* | *1H, 5,51* | *137,1 id* |
| * | 1H, 2.52 | * | 1H, 2.52 | **34** | 40,58 | 1H, 2,52 | 40,8 |
| 79.8 | 1H, 4.73 | 79.2 | 1H, 4.73 | **35** | 79,44 | 1H, 4,74 | 79,8 |
| 32.6 | 1.20-1.90 | 32.3 | 1H, 1.91 | **36** | 32,53 | 1H, 1,92 | 32,5 |
| 42.3 | 1.20-1.90 | 42.3 | 1.20-1.90 | **37** | 42,52 | 2H, 0,89/1,30 | 42,52 |
| 40.8 | 1.20-1.90 | 41.0 | 1.20-1.90 | **38** | 40,85 | 1H, 1,62 | 40,7 |
| 40.3 | 1.20-1.90 | 40.8 | 1.20-1.90 | **39** | 40,85 | 2H, 1,33/1,62 | 40,7 |
| 27.5 | 1.20-1.90 | 27.7 | 1.20-1.90 | **40** | 27,80 | 2H, 1,33/1,98 | 27,8 |
| 33.6 | 1H, 2.97 | 33.7 | 1H, 1.96 | **41** | 33,78 | 2H, 1,96 | 33,9 |
| *132.7 id* | *1H, 5.45* | *132.7 id* | *1H, 5.47* | ***42*** | *132, 72 id* | *1H, 5,48* | *133,0 id* |
| *129.8 id* | *1H, 5.43* | *129.8 id* | *1H, 5.42* | ***43*** | *129, 90 id* | *1H, 5,44* | *129,9 id* |
| 30.4 | 2H, 2.05 | 33.0 | 2H, 2.06/2.13 | **44** | 30,65 | 2H, 2,06 | 30,7 |
| 29.7 | 1.20-1.90 | 29.3 | 2H, 1.64 | **45** | 29,90 | 2H, 1,64 | 29,9 |
| 42.0 | 2H, 3.15 | 42.0 | 2H, 3.15 | **46** | 42,00 | 2H, 3,16 | 42,0 |
| 13.1 | 3H, 1.18 | 12.8 | 3H, 1.17 | **47** | 13,12 | 3H, 1,18 | 15,0 |
| 15.5 | 0.80-1.15 | 15.4 | 3H, 0.91 | **48** | 15,51 | 3H, 0,91 | 17,0 |
| 10.4 | 0.80-1.15 | 10.2 | 3H, 0.91 | **49** | 10,33 | 3H, 0,92 | 10,6 |
| 10.9 | 0.80-1.15 | 10.9 | 3H, 0.86 | **50** | 11,05 | 3H, 0,875 | 11,3 |
| 20.6 | 0.80-1.15 | 20.4 | 3H, 0.91 | **51** | 20,41 | 3H, 0,88 | 20,4 |
| 18.0 | 3H, 1.01 | 17.7 | 3H, 1.01 | **52** | 17,84 | 3H, 1,02 | 17,9 |
| 15.0 | 0.80-1.15 | 14.8 | 3H, 0.91 | **53** | 14,87 | 3H, 0,905 | 15,0 |
| 14.9 | 0.80-1.15 | 14.2 | 3H, 0.86 | **54** | 14,55 | 3H, 0,876 | 15,1 |
| 171.6 | - | 171.7 | - | **55** | 171,60 | - | 170,0 |
| 45.3 | 2H, 3.25 | 45.1 | 2H, 3.22 | **56** | 46,00 | 2H, 3,25 | 49,9 |
| 174.0 | - | 174.0 | - | **57** | 174,08 | - | 172,2 |
| 158.0 | - | 158.1 | - | **58** | 158,17 | - | 158,3 |
| *27.7 id* | *3H, 2.81* | *28.2 id* | *3H, 2.80* | ***59*** | *28,34 id* | *3H, 2,83* | *28,3 id* |
| | - | | - | **NH** | | 1H, 7,51 | - |
| 33.0 * | | 33.0* | | **60** | | | 169,5 |
| 33.4* | | 33.5* | | **61** | | | 44,5 |
| | | | | **62** | | | 171,8 |

### Summary of the NMR analysis:

1.1. Due to the high similarity or even identity of most of the ¹³C-NMR data the macrolactone ring with the guanidino side chain of the niphimycin family compounds discussed in here is assigned to be identical. The N-methyl group is indicated by the ¹H- and ¹³C-NMR-signals for all compounds.
1.2. The literature known 4,5-dihydroniphimycin shows three ¹³C-NMR signals in the range of δ_{c}=37.2-37.7. The compounds N-f12 and N-f13 show only one, resp. two, signals in the range of δ_{c}=37.2-37.7. The differences may occur due to differences in the positions of methyl or hydroxy substituents at the macrolactone ring.
All three compounds do not have a double bond at C4-C5 (δ_{c}=about 130 for double bonds), because the ¹³C-NMR signals appear in the range of δ_{c}=28-37 ppm.
1.3. The literature known malonylniphimycin (19-malonyl substituted niphimycin Iα) (and also niphimycin Iα (data not listed)) show another group of low field shift signals at about δ_{c}= 130) which clearly indicate an additional double bond.
1.4. The literature known 4,5-dihydroniphimycin shows two more ¹³C-NMR signals the range of δ_{c}=40-42. The new compounds N-f12 and N-f13 show two more ¹³C-NMR signals the range of δ_{c}=33. The differences may occur due to differences in the positions of methyl or hydroxy substituents at the macrolactone ring.
1.5. The ¹³C-NMR-data for the conjugated double bond at C-30-C-33 and for the double bond at C-42-C-43 are identical for the compounds 4,5-dihydroniphimycin, N-f12, N-f13 and malonylniphimycin (and also niphimycin Iα(data not listed)). This indicates the identical position of the double bond for all derivatives.

**Table 2: Comparison of the data from mass spectrometry analyis and UV spectra with the known 4,5-dihydroniphimycin and malonyl niphimycin**

| **Niphimycin Iα** | **N-f12** | **N-f13** | **4,5-Dihydro- niphimycin** *J. Antibiot.* **2000,** *53*, 627-632. | **Malonyl- niphimycin** *J. Antibiot.* **1997**, 50, 965-969 |
|---|---|---|---|---|
| **High resolution** | **High resolution** | **High resolution** | **High resolution** | **High resolution** |
| **MS:** [M+H]⁺= 1142.9 for C₅₉H₁₀₃N₃O₁₈ | **ESI-MS (FT-ICR):** [M+H]⁺ = 1144.7477 for C₅₉H₁₀₅N₃O₁₈ | **ESI-MS (FT-ICR):** [M+H]⁺ = 1144.7465 for C₅₉H₁₀₅N₃O₁₈ | **FAB-MS:** [M+H]⁺ = 1144.5200 for C₅₉H₁₀₅N₃O₁₈ | **FAB-MS:** [M+H]⁺ = 1228.9 for C₆₂H₁₀₅N₃O₂₁ |
| **Molecular weight:** 1141.9 | **Molecular weight:** 1143 | **Molecular weight:** 1143 | **Molecular weight:** 1143 | **Molecular weight:** 1227.9 |
| **UV spectra (abs. max.)** 228(sh), 232, 240(sh) | **UV spectra (abs. max.)** 231 | **UV spectra (abs. max.)** 231,239 | **UV spectra (abs. max.)** 225sh, 231, 241sh | **UV spectra (abs. max.)** 232 |

### Summary of the MS and UV analysis:

2.1. N-f12, N-f13 and the literature known 4,5-dihydroniphimycin show the identical high resolution MS peaks for the protonated molecular ion peak. They indicate the same molecular formular for the three compounds: C₅₉H₁₀₃N₃O₁₈.
2.2. The molecular formular of the new compounds N-f12 and N-f13 differ in 2 mass units from the parent compound of the niphimycin family niphimycin Iα and therefore clearly indicate N-f12 and N-f13 to be dihydroderivatives.
2.3. The UV spectra suggest all compound to belong to the niphimycin family.
2.4. For all compounds listed in here no significant fragment ions could be detected to support further the determined structures.

### Conclusion from the spectroscopic analysis:

The 4,5-dihydroniphimycin derivatives N-f12 and N-f13 appear to be both novel structures but the identity of one of them with the literature known 4,5-dihydroniphimycin can not be completely ruled out.
Concerning the position of the malonyl group, the methyl groups and hydroxy groups N-f12 and N-f13 can be regioisomers of the literature known 4,5-dihydroniphimycin.

### Example 3: Tyrosinase activity assay

Mammalian tyrosinases are found specifically in melanocytes, which are highly specialized cells located in the skin, hair bulbs and eyes and which produce pigments. They are key enzymes involved in the formation of melanin, which have a protective effect against the injury due to ultraviolet radiation. The copper-containing enzyme tyrosinase catalyzes the oxidation of the amino acid L-tyrosine into 3,4-dihydroxyphenylalanine (DOPA) and subsequently into DOPA-quinone. Further oxidation in eumelanogenesis/pheomelanogenesis leads to brown and black pigments depending on the incoporation of sulfur-containing reactants (e.g. cysteine or glutathione).
The here presented compounds N-f12 and N-f13 have been tested for their capability to interfere with the regulation of tyrosinase activity in cloudman S91 melanoma cells (ATCC-No. CCL 53.1). Briefly, S91 melanoma cells are seeded in microtiter plates and treated with test compounds for up to 48 hours at 37° C, 5 % CO₂, 95 % air and 100 % relative humidity (Hearing, V.J.;1987). In control cells tyrosinase activity is induced by addition of α-melanocortin stimulating hormone (α-MSH). After incubation the cells are lysed with a lysis buffer (50 mM potassium phophate; pH 6.8; 1% triton X-100™) and after centrifugation, the cleared supernatant is subsequently used for an enzymatic assay, e. g. the tyrosinase dependent oxidation of DOPA into DOPA-quinone, the latter beeing detectable at 475 nm in an automated plate reader.
As a general control the phsophodiesterase inhibitor IBMX (2-isobutyl-1-methylxanthine) was used, which leads to an accumulation of cAMP ― while blocking the phosphodiesterase dependent hydrolysation of cAMP - in the cytoplasm of IBMX treated cells in an MC1-R independent manner.
The compounds described in the present invention can be assayed for their influence on melanin biosynthesis employing the test described herein above. According to this test the bioactivity of these compounds are reported in figure 2.

### Example 4: cyclic AMP cell signaling interference

Regulation of gene expression by the second messenger cyclic AMP (cAMP) involves phosphorylation by protein kinase A (PKA) and activation of cAMP-response element binding protein (CREB)/CRE modulator (CREM) transcription factors which bind DNA to so called CRE response elements. One preferred pathway for the activation of the transcription factor CREB is its phosphorylation by PKA after stimulation of adenylyl cyclase activation by G-protein coupled receptor (GPCR) stimulation of the G_{S}-type receptors. As mentioned before the GPCR melanocortin 1 receptor - a main regulator of mammalian melanogenesis ― activates the adenylyl cyclase/protein kinase A (PKA) dependent intracellular signaling pathway. Therefore compounds interfering with this pathway should be able to render basal expression of a PKA/CREB-dependent signaling.
To analyze this activity, a CRE response element-dependent reporter gene assay was used. Briefly, the firefly luciferase gene under the control of a CREB-inducible basal SV40 promoter was used to transfect S91 melanoma cells. These cells respond well to natural ligands like α-MSH with enhanced melanin synthesis and therefore serve as a model system to study melanogenesis.
Given here as an example the above presented compounds N-f12 and N-f13 have the bioactive property to induce CRE-dependent luciferase expression in transfected S91 cells. For a typical experiment S91 cells were seeded at 2x10⁶ cells and transfected with 4 µg reporter gene in serum free medium by lipofection (lipofectamin plus; Invitrogen). After 3 - 6 hours the medium is replaced for a complete culture medium (RPMI, 2% fetal calf serum) and the next day the cells are seeded into 96 well plates at a density of 50000 cells/well. Stimulation of the transfected S91 cells takes place 24 hours after transfection for another 24 hours. In general the stimulation of transfected S91 melanoma cells leads to CRE-dependent reporter gene induction resulting in increased luciferase levels. After cell extraction (luciferase assay system, Promega), the luciferase activity was read out in an automated luminescence reader. As a general control the phsophodiesterase inhibitor IBMX (2-isobutyl-1-methylxanthine) was used, which leads to an accumulation of cAMP ― while blocking the phosphodiesterase dependent hydrolysation of cAMP - in the cytoplasm of IBMX treated cells in an MC1-R independent manner.
Figure 3 shows the bioactivity of the two compounds N-f12 and N-f13 in the CRE-dependent luciferase activity assay.

### Example 5: Tyrosinase activation in human primary melanocytes

As described before, mammalian tyrosinases are the key enzymes involved in melanogenesis and the formation of UV radiation protective pigments (eumelanin/pheomelanin).
The bioactivity of the here present compounds N-f12 and N-f13 on human melanogenesis was further tested in human primary cell culture experiments. For this purpose "Normal Human Epidermal Melanocytes" (NHEM) from foreskin (PromoCell GmbH / Heidelberg) of a five year old caucasian donor with brown eyes and hair and pale skin were employed. NHEM were grown at 37 °C, 5 % (v/V) CO₂ in steam saturated atmosphere in Melanocyte Growth Medium (PromoCell GmbH / Heidelberg; Catalog-No.: C-24010) containing 0.4 % bovine pituitary extract; 1 ng/ml basic fibroblast growth factor; 5 µg/ml insulin; 0,5 µg/ml hydrocortison; 10 ng/ml phorbol-myristate-acetate; 50 ng/ml amphotericin and 50 µg/ml gentamicin. For each passage cells were seeded at 10.000 cells per cm². To measure the tyrosinase activity of these primary melanocytes in the presence or absence of test compounds NHEMs were grown in Melanocyte Growth Medium (MGM) without bovine pituitary extract (PromoCell GmbH / Heidelberg; Catalog-No.: C-24110). Briefly, NHEMs were grown in MGM for 24 hours after cell passaging. For depletion of bovine pituitary extract (BPE) NHEMs were further cultivated for 72 hours in MGM without BPE. Following this cultivation procedure test compounds (200 nM α-MSH; 500 ng/ml N-f12/N-f13) were added for another 96 hours before human tyrosinase activity was assayed.
For the determination of tyrosinase activity cells were lysed in lysis buffer (50 mM potassium phosphate buffer; pH 6.8; 1% (v/v) triton X-100TM) and after centrifugation, the cleared supernatant is subsequently employed in an enzymatic assay, e. g. the tyrosinase dependent oxidation of DOPA into DOPA-quinone, the latter beeing detectable at 475 nm in an automated plate reader.

### References

Grabley, S. et al. (1990). Secondary metabolites by chemical screening. II. Amyxcins A and B, two novel niphimycin analogs isolated from a high producer strain of elaiophylin and nigericin. J. Anitbiotics. 43, 639-647.
Hearing, V. J. Jr. (1987) Monophenol monooxygenases (tyrosinase): purification, properties and reactions catalyzed. Methods Enzymol. 142, 154-165.
Ivanova, V. and Gushterova, A. (1979). Malonylniphimycin: Macrolide antibiotic from Streptomyces hygroscopicus B-7: Physico-chemical properties and structure elucidation. J. Antibiotics. 50(11). 965-969.
Ivanova, V. et al. (2000). Dihydroniphimycin: New Polyol macrolide antibiotic produced by Streptomyces hygroscopicus 15 Isolation and structure elucidation. J. Antibiotics. 53(6), 627-632.
Korn-Wendisch, F. and Kutzner, H.J. (1991) The Family Streptomycetaceae in: The Prokaryotes, 2nd. ed, Vol1, eds: Balows, A., Trüper, H.G., Dworkin, M., Harder, W. and Schleifer, K.-H., Springer-Verlag New York.
MacNeil, D. J. et al. (2002) The role of melanocortins in body weight regulation: opportunities for the treatment of obesity. Eur. J. Pharmacol. 440, 141-157.
Samama, P, et al. (2003) The melanocortin receptor MCR4 controls fat consumption. Regul. Pept. 113(1-3), 85-8.
Scott, M. C. et al (2002) Human melanocortin 1 receptor variants, receptor function and melanocyte response to UV radiation. Journal of Cell Science 115(11), 2349-2355.
Stefanell, S. (1997) Inhibitors of type-I Interleukin-1 receptor from microbial metabolites. Journal of Antibiotics. 50(6), 484-489.**Strunk et al. (1997)** ARB: a software environment for sequence data. www.mikro.biologie.tu-muenchen.de/pub/ARB
Suzuki, I. et al. (1996) Binding capacitiy and activation of the MCI receptors by melanotropic hormones correlate directly with their mitogenic and melanogenic effects on human melanocytes.Endocrinology 137, 1627-1633.

## Claims

1. A method of producing a compound having activity in a cell-based tyrosinase assay, the method comprising
(a) culturing *Streptomyces hygroscopicus subsp. hygroscopicus* (DSMZ deposit number DSM 40578);
(b) separating the medium containing the compound from the bacterial cells;
(c) solid phase extraction of said medium, thereby obtaining a crude extract;
(d) performing gel filtration of said crude extract;
(e) taking a gel filtration fraction exhibiting activity in said assay;
(f) performing HPLC of said fraction exhibiting activity in said assay;
(g) taking one or more HPLC fractions exhibiting activity in said assay, thereby obtaining said compound; and
(h) testing endogenous tyrosinase induction,
wherein said assay comprises determining formation of DOPA-quinone from tyrosine or DOPA.

2. Use of a compound represented by the general formula (I) 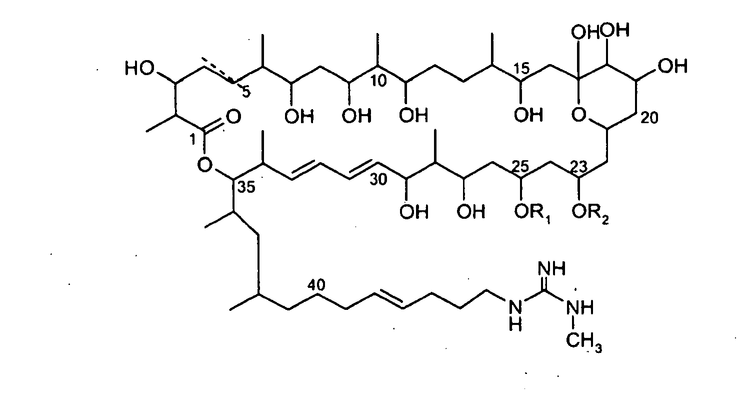 wherein the bond between C₄ and C₅ is either a single bond or a double bond,
wherein
(i) R₁ = H and R₂ = -COCH₂COOH; or
(ii) R₁ = -COCH₂COOH and R₂ = H.
In a cosmetic composition, wherein said composition is to be administered to the skin, and a tanned skin is brought about by the administration and/or sun protection is provided thereby.

3. The use of claim 2, wherein the bond between C₄ and C₅ is a single bond.

4. The use of claim 2 or 3, wherein the cosmetic composition is to be applied to skin, hair and/or eyes of a human subject or of an animal.

5. The use of any one of claims 2 to 4, wherein the application of the cosmetic composition reduces freckles and age spots of the body part to which said cosmetic composition is applied to.

6. A compound represented by the general formula (I) as defined in claim 2 for use in the treatment of pigmentation disorders.

7. The compound for use according to claim 6, wherein the pigmentation disorder is selected from vitiligo and albinism, such as oculocutaneous albinism type 1, 2 or 3.

8. A compound represented by formula (II) 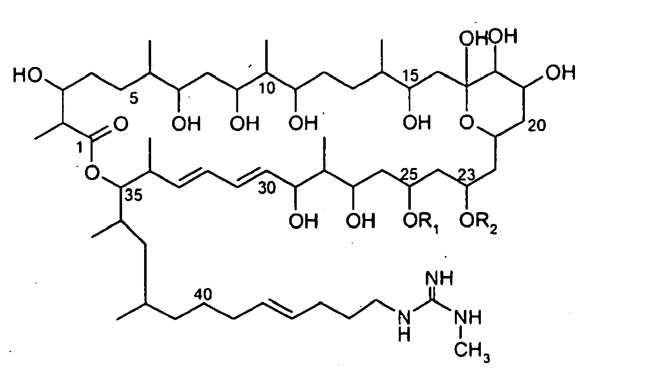 wherein R₁ = -COCH₂COOH and R₂ = H.

9. A pharmaceutical or cosmetic composition comprising either the compound of claim 8 or a compound represented by formula (III) 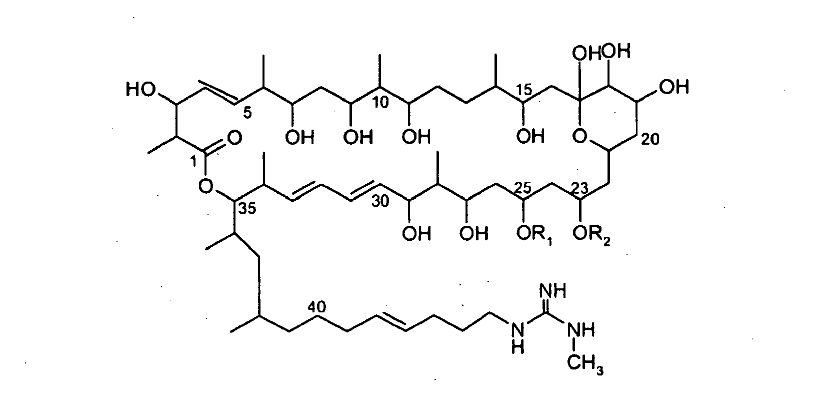 wherein R₁ = -COCH₂COOH and R₂ = H.

## Patentansprüche

1. Verfahren zur Herstellung einer Verbindung, die Aktivität in einem auf Zellen basierenden Tyrosinase-Assay hat, wobei das Verfahren umfasst:
(a) Züchten von *Streptomyces hygroscopicus subsp. hygroscopicus* (DSMZ-Hinterlegungsnummer DSM 40578);
(b) Abtrennen des Mediums, das die Verbindung enthält, von den bakteriellen Zellen;
(c) Festphasenextraktion des Mediums, wodurch ein Rohextrakt erhalten wird;
(d) Ausführen einer Gelfiltration des Rohextrakts;
(e) Entnahme einer Fraktion der Gelfiltration, die Aktivität im Assay zeigt;
(f) Ausführen einer HPLC mit der Fraktion, die Aktivität im Assay zeigt;
(g) Entnahme einer oder mehrerer HPLC-Fraktionen, die Aktivität im Assay zeigen, wodurch die Verbindung erhalten wird; und
(h) Testen der endogenen Tyrosinase-Induktion,
wobei der Assay die Bestimmung der Bildung von DOPA-Chinon aus Tyrosin oder DOPA umfasst.

2. Verwendung einer Verbindung, die durch die allgemeine Formel (I) dargestellt ist 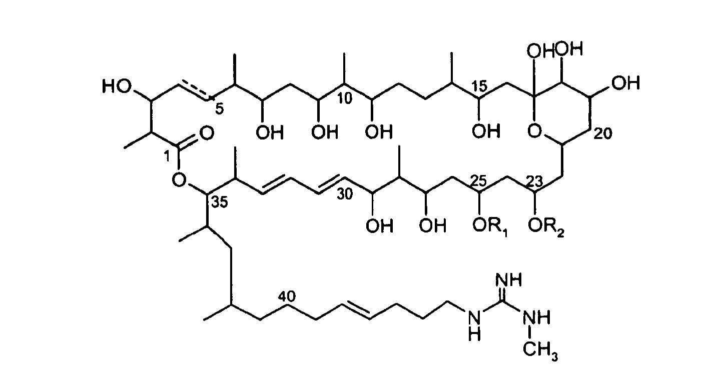 wobei die Bindung zwischen C₄ und C₅ entweder eine Einzelbindung oder eine Doppelbindung ist, wobei
(i) R₁ = H und R₂ = -COCH₂COOH; oder
(ii) R₁ = -COCH₂COOH und R₂ = H,
in einer kosmetischen Zusammensetzung, wobei die Zusammensetzung auf die Haut verabreicht werden soll und durch die Verabreichung eine gebräunte Haut bewirkt wird und/oder hierdurch Sonnenschutz verliehen wird.

3. Verwendung nach Anspruch 2, wobei die Bindung zwischen C₄ und C₅ eine Einzelbindung ist.

4. Verwendung nach Anspruch 2 oder 3, wobei die kosmetische Zusammensetzung auf die Haut, die Haare und/oder die Augen eines Menschen oder eines Tieres aufgebracht wird.

5. Verwendung nach einem der Ansprüche 2 bis 4, wobei das Aufbringen der kosmetischen Zusammensetzung Sommersprossen und Altersflecken auf den Körperteilen reduziert, auf die die kosmetische Zusammensetzung aufgebracht wird.

6. Verbindung, die durch die allgemeine Formel (I) wie in Anspruch 2 definiert dargestellt ist, zur Verwendung in der Behandlung von Pigmentierungsstörungen.

7. Verbindung zur Verwendung nach Anspruch 6, wobei die Pigmentierungsstörung aus der Weißfleckenkrankheit und Albinismus, wie oculocutaner Albinismus Typ 1, 2 oder 3, ausgewählt ist.

8. Verbindung, die durch die Formel (II) dargestellt ist 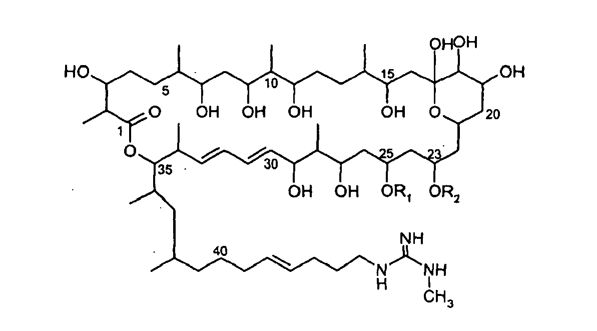 wobei R₁ = -COCH₂COOH und R₂ = H.

9. Pharmazeutische oder kosmetische Zusammensetzung, die entweder die Verbindung nach Anspruch 8 oder eine Verbindung, die durch die Formel (III) dargestellt ist, umfasst 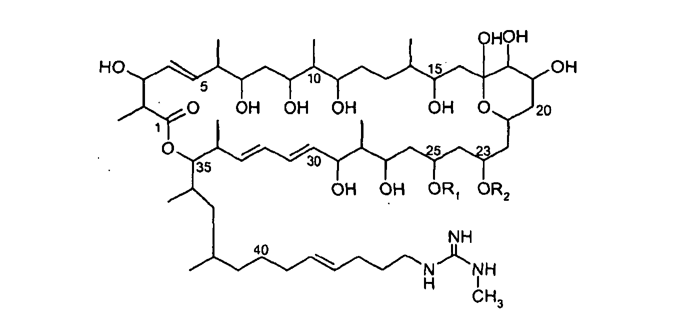 wobei R₁ = -COCH₂COOH und R₂ = H.

## Revendications

1. Méthode pour produire un composé ayant une activité dans un test cellulaire de tyrosinase, la méthode comprenant:
(a) la culture de *Streptomyces hygroscopicus subsp. hygroscopicus* (numéro de dépôt DSMZ : DSM 40578) ;
(b) la séparation du milieu contenant le composé à partir des cellules bactériennes ;
(c) l'extraction en phase solide dudit milieu, obtenant ainsi un extrait brut ;
(d) la réalisation d'une filtration sur gel dudit extrait brut ;
(e) la prise d'une fraction de filtration sur gel montrant une activité dans ledit test ;
(f) la réalisation d'une HPLC de ladite fraction montrant une activité dans ledit test;
(g) la prise d'une ou plusieurs fractions HPLC montrant une activité dans ledit test, obtenant ainsi ledit composé ; et .
(h) le test d'induction de tyrosinase endogène,
dans laquelle ledit test comprend la détermination de la formation de DOPA-quinone à partir de tyrosine ou DOPA.

2. Utilisation d'un composé représenté par la formule générale (I) 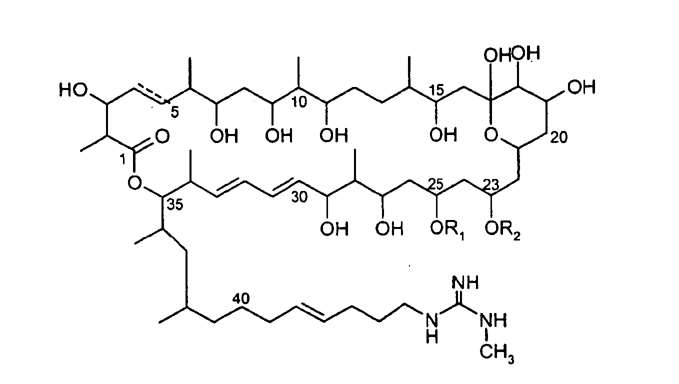 dans laquelle la liaison entre C₄ et C₅ est soit une liaison simple soit une liaison double, où
(i) R₁ = H et R₂ = -COCH₂COOH ; ou
(ii) R₁ = -COCH₂COOH et R₂ = H
dans une composition cosmétique, ladite composition étant destinée à être administrée sur la peau, et une peau bronzée est provoquée par l'administration et/ou la protection solaire est assurée de ce fait.

3. Utilisation selon la revendication 2, dans laquelle la liaison entre C₄ et C₅ est une liaison simple.

4. Utilisation selon la revendication 2 ou 3, dans laquelle la composition cosmétique est destinée à être appliquée sur la peau, les cheveux et/ou les yeux d'un sujet humain ou d'un animal.

5. Utilisation selon l'une quelconque des revendications 2 à 4, dans laquelle l'application de la composition cosmétique réduit les taches de rousseur et les taches d'âge de la partie du corps sur laquelle ladite composition cosmétique est appliquée.

6. Composé représenté par la formule générale (I) comme définie dans la revendication 2 pour utilisation dans le traitement des désordres de pigmentation.

7. Composé pour utilisation selon la revendication 6, où le désordre de pigmentation est choisi parmi le vitiligo et l'albinisme, tel que l'albinisme oculocutané de type 1, 2 ou 3.

8. Composé représenté par la formule (II) 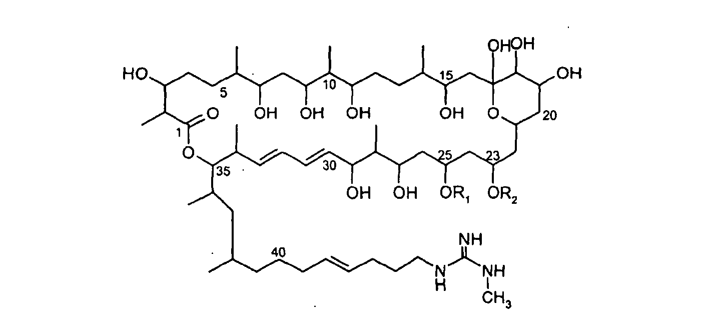 dans laquelle R₁ = -COCH₂COOH et R₂ = H.

9. Composition pharmaceutique ou cosmétique comprenant soit le composé de la revendication 8 soit un composé représenté par la formule (III) 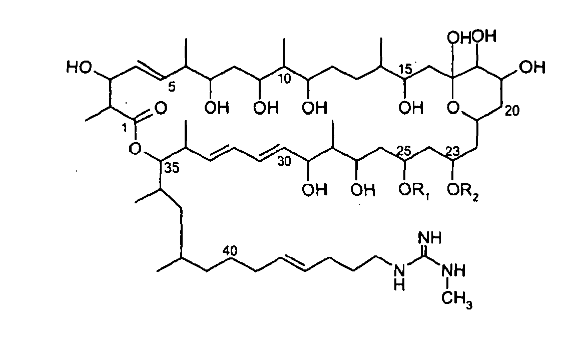 dans laquelle R₁ = -COCH₂COOH et R₂ = H.
